# EUROPÄISCHE PATENTANMELDUNG

(11) **EP 1 967 180 A1**
(43) Veröffentlichungstag der Anmeldung: **10.09.2008**
(21) Anmeldenummer: 07004537.2
(22) Anmeldetag: 06.03.2007
(51) Int. Cl.: A61K 9/06, A61K 9/107, A61K 47/24, A61K 47/36, A61K 47/38, A61K 31/07, A61K 31/203, A61K 31/192, A61K 31/165

(54) **Topische Zusammensetzung enthaltend einen Retinoid-Rezeptor-Agonist**

(71) Anmelder: Almirall Hermal GmbH, 21465 Reinbek (DE)
(72) Erfinder: Fielhauer, Sabine, 21481 Lauenburg (DE); Evers, Fritjof, 21465 Reinbek (DE); Treudler, Klaus, Dr., 21217 Seevetal (DE); Mallwitz, Henning, Dr., 21244 Buchholz (DE)
(74) Vertreter: UEXKÜLL & STOLBERG

(57) **Zusammenfassung**

Die Erfindung betrifft eine topische Zusammensetzung, die (a) Retinoid-Rezeptor-Agonist, (b) Gelbildner und (c) Phospholipid enthält. Die Erfindung betrifft auch die Verwendung der topischen Zusammensetzung zur Behandlung von Hauterkrankungen.

## Beschreibung

Die Erfindung betrifft eine topische Zusammensetzung, die einen Retinoid-Rezeptor-Agonist enthält und insbesondere zur Behandlung von Hauterkrankungen wie Akne eingesetzt werden kann.

Acne vulgaris ist weltweit die häufigste dermatologische Erkrankung. Fast alle Jugendlichen im Alter von 12 bis 17 Jahren sind von Akneerkrankungen betroffen. Allerdings leiden manche Patienten auch noch im fortgeschrittenen Erwachsenenalter an diesem Krankheitsbild.

Für die Entstehung von Akne gibt es mehrere Ursachen. Zu diesen Ursachen gehören übermäßige Talgproduktion; Proliferation von Bakterien wie *Propionibacterium acnes,* Entzündungen sowie die mit dem Beginn der Pubertät zusammenhängende Zunahme der Produktion von Androgenen, die zu übermäßiger Abschilferung follikulärer Korneozyten führt.

Die Therapie der Akne wird individuell an die Schwere und den Typ der Akneerkrankung nach Lokalisation und Hauttyp angepasst. Zur Behandlung von leichter und mittelschwerer Akne wird im Allgemeinen eine topische Medikation empfohlen.

Verschiedene Wirkstoffe für die Behandlung von Akne sind bekannt. So werden beispielsweise zur Behandlung bestimmter Formen von Akne Retinoid-Rezeptor-Agonisten wie Adapalen verwendet. Biochemische und pharmakologische Studien haben gezeigt, dass Adapalen ein Modulator der Zelldifferenzierung, der Keratinisierung und entzündlicher Prozesse ist. Adapalen bindet an spezifische Retinolsäure-Rezeptoren. Obwohl die genaue Wirkungsweise des Adapalens nicht bekannt ist, wird davon ausgegangen, dass topisch appliziertes Adapalen die Differenzierung follikulärer Epithelzellen normalisiert, was zu einer verminderten Mikrokomedonbildung führt. Klinische Studien haben gezeigt, dass Adapalen ein wirksames Therapeutikum zur Behandlung von leichter und mittelschwerer Acne vulgaris ist.

Ein anderer in der Behandlung von Akne eingesetzter Wirkstoff ist Benzoylperoxid (BPO). Benzoylperoxid wird seit langem in der Dermatologie zur Behandlung von Acne vulgaris verwendet. Der therapeutische Effekt von Benzoylperoxid bei Akne scheint auf der Verminderung der Bakterienflora, z.B. von *Propionibacterium acnes,* sowie seiner leichten sebostatischen und keratolytischen Wirkung zu beruhen. Benzoylperoxid ist viele Jahrzehnte lang in Konzentrationen bis zu 10 % angewendet worden.

WO 03/55472 beschreibt eine Gelzusammensetzung zur Verhinderung oder Behandlung verschiedener Störungen einschließlich Akne, die eine Formulierung von mindestens einem Retinoid, dispergiertem Benzoylperoxid und mindestens einem pHunabhängigen Gelbildner in einem physiologisch annehmbaren Medium enthält.

Allerdings hat sich herausgestellt, dass eine steigende Zahl von Akne-Patienten zumindest teilweise trockene Haut hat. Zudem empfinden viele Patienten Anti-Akne-Wirkstoffe wie Adapalen oder Benzoylperoxid als hautaustrocknend.

Es besteht daher ein Bedarf an einer Zusammensetzung zur Behandlung von Hauterkrankungen wie Akne, die bei topischer Applikation Hautaustrocknung minimiert und hautaustrocknenden Eigenschaften von Anti-Akne-Wirkstoffen entgegenwirkt.

Diese Aufgabe wird überraschenderweise durch die Zusammensetzung gemäß den Ansprüchen 1 bis 12 gelöst. Die Erfindung betrifft außerdem die Verwendung der Zusammensetzung gemäß den Ansprüchen 13 und 14.

Die erfindungsgemäße topische Zusammensetzung zeichnet sich dadurch aus, dass sie
a) Retinoid-Rezeptor-Agonist,
b) Gelbildner und
c) Phospholipid
enthält.

In der erfindungsgemäßen Zusammensetzung wird ein Gelbildner als Gelkomponente mit einem Phospholipid als Lipidkomponente kombiniert. Durch die Kombination der Gelkomponente und der Lipidkomponente wird eine Quasi-Emulsion gebildet. In dieser ist mindestens ein Retinoid-Rezeptor-Agonist enthalten, welcher bevorzugt als Anti-Akne-Wirkstoff fungiert.

Die erfindungsgemäße Zusammensetzung stellt eine neue Formulierung dar, bei der überraschenderweise bestimmte Eigenschaften von Gelen und Cremes so kombiniert werden konnten, dass eine verbesserte Eignung zur Behandlung von Hauterkrankungen wie Akne bei einer breiten Patientenpopulation erreicht wird.

Insbesondere wird durch den Creme-Anteil der Formulierung die Austrocknung der Haut minimiert und eine durch Anti-Akne-Wirkstoffe hervorgerufene Verschlechterung des Zustands trokkener Haut vermieden. Zugleich wird durch den Hydrogel-Anteil der Formulierung und die Verwendung von Phospholipid im Creme-Anteil vermieden, der Hautoberfläche übermäßig Fett zuzuführen. Dadurch wird auch verhindert, dass nach der Anwendung ein fettiges Hautgefühl zurückbleibt. Dies ist auch deshalb wichtig, weil viele Akneerkrankungen mit übermäßiger Talgproduktion einhergehen. Außerdem ist vor allem bei entzündlicher Akne auch der kühlende Effekt von Gelen vorteilhaft.

Die erfindungsgemäße Zusammensetzung hat zudem gute kosmetische Eigenschaften und eine hohe Akzeptanz bei den Patienten.

Bevorzugt ist eine Zusammensetzung, bei der der Retinoid-Rezeptor-Agonist Adapalen oder ein Retinoid ist. Dabei sind Tretinoin, Isotretinoin, Motretinid, Tazaroten und/oder Retinol besonders bevorzugte Retinoide. Ganz besonders bevorzugt ist der Retinoid-Rezeptor-Agonist Adapalen. Es ist zudem bevorzugt, dass die Zusammensetzung 0,01 - 1,0 Gew.-%, besonders bevorzugt 0,025 - 0,3 Gew.-% Retinoid-Rezeptor-Agonist enthält.

Die erfindungsgemäße Zusammensetzung enthält mindestens einen Gelbildner. Der Begriff "Gelbildner" bezeichnet hier einen Bestandteil der Zusammensetzung, der in einer wässrigen Phase eine aus kolloidalen Suspensionen bestehende viskoelastische Masse bildet. Zur Verwendung in der erfindungsgemäßen Zusammensetzung sind verschiedene Gelbildner geeignet. Dabei ist eine Zusammensetzung bevorzugt, die mindestens einen Gelbildner ausgewählt aus der Gruppe bestehend aus Celluloseethern, Xanthangummi, Carbomeren, Alginaten und Magnesium-Silikat enthält. Von diesen sind Carbomere besonders bevorzugt.

Beispiele für geeignete Celluloseether sind Alkylcellulosen, wie Methylcellulose und Hydroxyethylcellulose.

Carbomere sind insbesondere mit Polyalkenyl-Polyether quervernetzte Homo- oder Copolymere der Acrylsäure. Zu Beispielen für geeignete Carbomere gehören Carbomer 934, Carbomer 940 (Synthalen K) und Carbomer 941.

Alginate sind Salze oder Ester der Alginsäure. Beispiele für geeignete Alginate sind Natrium-Alginat und PropylenglycolAlginat.

Die gewünschte Gelbildung wird in Abhängigkeit von der Art des Gelbildners durch unterschiedliche Mengen des Gelbildners erreicht. Eine geeignete Gelbildung kann beispielsweise dadurch erreicht werden, dass die Zusammensetzung
0,1 - 5,0 Gew.-%, bevorzugt 0,5 - 2,5 Gew.-% Celluloseether;
0,1 - 5,0 Gew.-%, bevorzugt 0,5 - 2,5 Gew.-% Xanthangummi;
0,1 - 3,0 Gew.-%, bevorzugt 0,4 - 2,0 Gew.-% Carbomer;
0,1 - 5,0 Gew.-%, bevorzugt 0,5 - 2,5 Gew.-% Alginat; und/oder
0,1 - 8,0 Gew.-%, bevorzugt 0,5 - 5,0 Gew.-% Magnesium-Silikat
enthält.

Besonders bevorzugt ist eine Zusammensetzung, die 0,2 - 5,0 Gew.-%, weiter bevorzugt 0,5 - 2,5 Gew.-%, und ganz besonders bevorzugt 1,0 - 2,0 Gew.-% Gelbildner enthält.

Die erfindungsgemäße Zusammensetzung enthält ein Phospholipid. Dabei ist es bevorzugt, dass das Phospholipid ein Phosphoglycerid, besonders bevorzugt nicht-hydriertes oder hydriertes Phosphatidylcholin ist. Bevorzugt enthält die Zusammensetzung eine Lecithinfraktion mit hohen Anteilen an nicht-hydriertem und/oder hydriertem Phosphatidylcholin. Geeignete Lecithinfraktionen sind beispielsweise unter den Namen Phospholipon^{®} 90 NG (nicht-hydriert) oder Phospholipon^{®} 80 H (hydriert) von der Phospholipid GmbH erhältlich. Weiter ist es bevorzugt, dass die Zusammensetzung 1,0 - 10,0 Gew.-%, besonders bevorzugt 2,0 - 7,5 Gew.-% Phospholipid enthält.

Die erfindungsgemäße Zusammensetzung kann weitere Bestandteile enthalten.

In einer bevorzugten Ausführungsform enthält die Zusammensetzung mindestens einen nicht-ionischen Emulgator. Beispiele für geeignete nicht-ionische Emulgatoren sind ethoxylierte Zuckeralkoholfettsäureester, wie Polysorbat 80, und (gemischte) Polyalkylenglykole, wie Poloxamere. Es ist weiterhin bevorzugt, dass die erfindungsgemäße Zusammensetzung 0,2 - 2,0 Gew.-%, besonders bevorzugt 0,5 - 1,0 Gew.-% nicht-ionischen Emulgator enthält.

In einer anderen Ausführungsform ist die erfindungsgemäße Zusammensetzung im Wesentlichen frei von zusätzlichen Emulgatoren.

In einer weiteren bevorzugten Ausführungsform enthält die erfindungsgemäße Zusammensetzung mindestens ein Polyol. Beispiele für geeignete Polyole sind mehrwertige Alkanole, wie Glykole oder Glycerin, und lineare oder cyclische Zuckeralkohole, wie Sorbit oder Inosit. Ein besonders bevorzugtes Polyol ist 1,3-Butylenglykol. Polyole als Bestandteil der erfindungsgemäßen Zusammensetzung können als Feuchthaltemittel wirken und damit den feuchtigkeitsspendenden Effekt der Formulierung verstärken. Es ist weiter bevorzugt, dass die erfindungsgemäße Zusammensetzung 2,0 - 20,0 Gew.-%, besonders bevorzugt 5,0 - 15,0 Gew.-% Polyol enthält.

Erfindungsgemäße Zusammensetzungen können gegebenenfalls auch weitere Wirkstoffe, wie weitere Anti-Akne-Wirkstoffe enthalten. Beispiele für geeignete Anti-Akne-Wirkstoffe sind a-Hydroxy-Säuren, Salicylsäure, Antibiotika und Azelainsäure. Kombinationsprodukte haben den Vorteil, dass sie aufgrund ihrer verbesserten Effizienz und der damit zusammenhängenden höheren Compliance zu besseren Behandlungsergebnissen und somit zu einer höheren Akzeptanz bei den Patienten führen können. Ein besonders geeigneter weiterer Anti-Akne-Wirkstoff ist Benzoylperoxid.

Insbesondere bei Anwendung höherer Dosierungen von Benzoylperoxid in den bisher bekannten Formulierungen von Anti-Akne-Mitteln haben allerdings viele Patienten über Hautaustrocknung geklagt. Es ist daher ein besonderer Vorteil der erfindungsgemäßen Zusammensetzung, dass diese auch in einem Kombinationsprodukt, das einen Retinoid-Rezeptor-Agonisten und Benzoylperoxid enthält, eine Austrocknung der Haut weitgehend verhindern oder zumindest deutlich reduzieren kann.

In einer bevorzugten Ausführungsform enthält die erfindungsgemäße Zusammensetzung daher als weiteren Anti-Akne-Wirkstoff Benzoylperoxid. Es ist weiter bevorzugt, dass die Zusammensetzung 0,5 -15,0 Gew.-%, besonders bevorzugt 2,5 - 7,5 Gew.-% Benzoylperoxid enthält.

Es hat sich überraschend gezeigt, dass eine erfindungsgemäße Zusammensetzung auch dann stabil ist, wenn sie als Gelbildner z.B. Carbomer und als weiteren Anti-Akne-Wirkstoff Benzoylperoxid enthält. Dabei kann der Einfluss von durch Zersetzung des Benzoylperoxids freigesetzter Benzoesäure auf die Stabilität der Gelphase überraschenderweise durch die Verwendung ausreichend hoher Mengen an Carbomer innerhalb der oben angegebenen Mengenbereiche kompensiert werden.

Die erfindungsgemäße Zusammensetzung kann auch eine Base oder ein Puffermittel enthalten, um die Zusammensetzung auf einen gewünschten pH-Wert einzustellen. Beispiele für geeignete Basen sind anorganische Basen, wie Alkali- oder Erdalkalimetallhydroxide, oder organische Basen, wie primäre, sekundäre oder tertiäre Amine oder quartäre organische Ammoniumhydroxide. Besonders bevorzugte Basen sind Alkalimetallhydroxide, wie Natriumhydroxid.

Die erfindungsgemäße Zusammensetzung sollte einen zur topischen Applikation geeigneten, vorzugsweise einen hautfreundlichen pH-Wert haben. Es ist bevorzugt, dass die Zusammensetzung einen pH-Wert im Bereich von 3,5 - 9,0, besonders bevorzugt 5,0 - 7,5, ganz besonders bevorzugt 6,0 - 6,5 aufweist.

Schließlich enthält die erfindungsgemäße Zusammensetzung üblicherweise Wasser. Es ist bevorzugt, dass die Zusammensetzung 50,0 - 90,0 Gew.-%, besonders bevorzugt 70,0 - 85,0 Gew.-% Wasser enthält.

Die Erfindung betrifft auch die Verwendung der erfindungsgemäßen Zusammensetzung zur Behandlung von Hauterkrankungen. Dabei ist die Hauterkrankung bevorzugt Akne, besonders bevorzugt Akne vulgaris.

Die Erfindung wird im folgenden anhand von Beispielen näher erläutert.

### Beispiele:

### Beispiele 1 bis 7:

Zur Herstellung erfindungsgemäßer Zusammensetzungen wurde wie folgt vorgegangen:

Die Komponenten Polysorbat 80, Phospholipid und 40 Gew.-% des Butandiols wurden zusammen auf etwa 70 °C erwärmt. Diese Phase wurde mit etwa 40 Gew.-% der angegebenen Menge Wasser emulgiert. Adapalen wurde in 10 Gew.-% des Butandiols suspendiert. Diese Suspension wurde zur Phospholipid-Phase gegeben.

Die Restmenge Wasser wurde mit der Restmenge Butandiol vermischt. In dieser Mischung wurde das Benzoylperoxid sorgfältig dispergiert. Diese Suspension wurde auf einer Mühle vermahlen, bis die Teilchengröße des Benzoylperoxids weniger als 100 µm betrug. Zu dieser feinen Benzoylperoxid-Suspension wurde der Gelbildner (Methylcellulose, Xanthangummi oder Carbomer) gegeben und klümpchenfrei verteilt. Gegebenenfalls wurde diese Phase anschließend mit Natriumhydroxid neutralisiert.

Das entstehende Gel wurde mit der Phospholipid-Phase sorgfältig vermischt.

Die dabei entstehenden Produkte hatten die folgende Zusammensetzung (in Gew.-%):

### Beispiel 1:

| | |
|---|---|
| Adapalen | 0,05 |
| Benzoylperoxid | 1,00 |
| Polysorbat 80 | 0,75 |
| 1,3-Butandiol | 8,00 |
| Methylcellulose | 2,00 |
| Hydriertes Phospholipid (Phospholipon 80 H) | 3,00 |
| Wasser, gereinigt | 85,20 |

### Beispiel 2:

| | |
|---|---|
| Adapalen | 0,10 |
| Benzoylperoxid | 1,00 |
| Polysorbat 80 | 0,75 |
| 1,3-Butandiol | 8,00 |
| Xanthangummi | 2,00 |
| Phospholipid (Phospholipon 90 NG) | 7,00 |
| Wasser, gereinigt | 81,15 |

### Beispiel 3:

| | |
|---|---|
| Adapalen | 0,20 |
| Benzoylperoxid | 2,50 |
| Polvsorbat 80 | 0,75 |
| 1,3-Butandiol | 8,00 |
| Carbomer 934 | 1,75 |
| Natriumhydroxid | 1,50 |
| Phospholipid (Phospholipon 90 NG) | 3,00 |
| Wasser, gereinigt | 82,30 |

### Beispiel 4:

| | |
|---|---|
| Adapalen | 0,10 |
| Benzoylperoxid | 5,00 |
| Polysorbat 80 | 0,75 |
| 1,3-Butandiol | 8,00 |
| Carbomer 940 (Synthalen K) | 1,75 |
| Natriumhydroxid | 1,50 |
| Hydriertes Phospholipid (Phospholipon 80 H) | 3,00 |
| Wasser, gereinigt | 79,90 |

### Beispiel 5:

| | |
|---|---|
| Adapalen | 0,30 |
| Benzoylperoxid | 5,00 |
| Polysorbat 80 | 0,75 |
| 1,3-Butandiol | 8,00 |
| Carbomer 941 | 1,75 |
| Natriumhydroxid | 1,50 |
| Phospholipid (Phospholipon 90 NG) | 7,00 |
| Wasser, gereinigt | 75,70 |

### Beispiel 6:

| | |
|---|---|
| Adapalen | 0,10 |
| Benzoylperoxid | 7,50 |
| Polysorbat 80 | 0,75 |
| 1,3-Butandiol | 8,00 |
| Carbomer 940 (Synthalen K) | 1,75 |
| Natriumhydroxid | 1,50 |
| Hydriertes Phospholipid (Phospholipon 80 H) | 3,00 |
| Wasser, gereinigt | 77,40 |

### Beispiel 7:

| | |
|---|---|
| Adapalen | 0,10 |
| Benzoylperoxid | 10,00 |
| Polysorbat 80 | 0,75 |
| 1,3-Butandiol | 8,0 |
| Carbomer 940 (Synthalen K) | 1,75 |
| Natriumhydroxid | 1,50 |
| Hydriertes Phospholipid (Phospholipon 80 H) | 3,00 |
| Wasser, gereinigt | 74,90 |

## Patentansprüche

1. Topische Zusammensetzung, die
(a) Retinoid-Rezeptor-Agonist,
(b) Gelbildner und
(c) Phospholipid
enthält.

2. Zusammensetzung nach Anspruch 1, bei der der Retinoid-Rezeptor-Agonist Adapalen oder ein Retinoid, bevorzugt Tretinoin, Isotretinoin, Motretinid, Tazaroten und/oder Retinol ist.

3. Zusammensetzung nach Anspruch 1 oder 2, bei der der Gelbildner ein Celluloseether, Xanthangummi, ein Carbomer, ein Alginat und/oder ein Magnesium-Silikat ist.

4. Zusammensetzung nach einem der Ansprüche 1 bis 3, bei der das Phospholipid ein Phosphoglycerid, bevorzugt nicht-hydriertes und/oder hydriertes Phosphatidylcholin ist.

5. Zusammensetzung nach einem der Ansprüche 1 bis 4, die 0,01 - 1,0 Gew.-%, bevorzugt 0,025 - 0,3 Gew.-% Retinoid-Rezeptor-Agonist enthält.

6. Zusammensetzung nach einem der Ansprüche 1 bis 5, die 0,2 - 3,0 Gew.-%, bevorzugt 0,5 - 2,5 Gew.-%, besonders bevorzugt 1,0 - 2,0 Gew.-% Gelbildner enthält.

7. Zusammensetzung nach einem der Ansprüche 1 bis 6, die 1,0 - 10,0 Gew.-%, bevorzugt 2,0 - 7,5 Gew.-% Phospholipid enthält.

8. Zusammensetzung nach einem der Ansprüche 1 bis 7, die 0,2 - 2,0 Gew.-%, bevorzugt 0,5 - 1,0 Gew.-% nicht-ionischen Emulgator enthält.

9. Zusammensetzung nach einem der Ansprüche 1 bis 8, die 2,0 - 20,0 Gew.-%, bevorzugt 5,0 - 15,0 Gew.-% Polyol enthält.

10. Zusammensetzung nach einem der Ansprüche 1 bis 9, die 50,0 - 90,0 Gew.-%, bevorzugt 70,0 - 85,0 Gew.-% Wasser enthält.

11. Zusammensetzung nach einem der Ansprüche 1 bis 10, die 0,5 - 15,0 Gew.-%, bevorzugt 2,5 - 7,5 Gew.-% Benzoylperoxid enthält.

12. Zusammensetzung nach einem der Ansprüche 1 bis 11, die einen pH-Wert im Bereich von 3,5 - 9,0, bevorzugt 5,0 - 7,5, besonders bevorzugt 6,0 - 6,5 aufweist.

13. Verwendung der Zusammensetzung nach einem der Ansprüche 1 bis 12 zur Behandlung von Hauterkrankungen.

14. Verwendung nach Anspruch 13, bei der die Hauterkrankung Akne, bevorzugt Akne vulgaris ist.
